# EUROPEAN PATENT SPECIFICATION

(11) **EP 0 448 540 B1**
(45) Date of publication and mention of the grant of the patent: **22.11.1995**
(21) Application number: 91870043.6
(22) Date of filing: 18.03.1991
(51) Int. Cl.: C07D 213/803, A01N 43/40

(54) **Process for preparation of fluoromethyl-substituted pyridine dicarboxylates**
Verfahren zur Herstellung von Fluoromethyl-substituierten Pyridindicarboxylaten
Procédé pour la préparation de pyridine dicarboxylates, fluorométhyl substitués

(30) Priority: 19.03.1990 US 495185; 19.03.1990 US 495174
(43) Date of publication of application: 25.09.1991
(73) Proprietor: ROHM AND HAAS COMPANY, Philadelphia, Pennsylvania 19106-2399 (US)
(72) Inventor: Miller, William Harold, Glendale, Missouri 63122 (US); Pulwer, Mitchell Joel, St. Louis, Missouri 63146 (US)
(74) Representative: Tanner, James Percival

(56) References cited:
- EP-A- 0 133 612
- JOURNAL OF THE CHEMICAL SOCIETY, CHEMICAL COMMUNICATIONS. no. 17, September 1989, LETCHWORTH GB pages 1279 - 1280; M. OLWEGARD: 'Preferance for anti 1,4-elimination of hydrochloric acid promoted by base in systems with non-cyclic c819-c829 bonds'

## Description

Methods for preparation of 2,6-bis(fluorinated methyl)-pyridine dicarboxylates and pyridine dicarbothioates are disclosed in U.S. Patents 4,692,184 and 4,618,679 and in European Patent 135,491. These compounds are useful as herbicides.

### DESCRIPTION OF THE PRIOR ART

As used herein, the following terms have the following meanings:
DABCO - 1,4-diazabicyclo-[2.2.2]-octane
DBU - 1,8-diazabicyclo-[5.4.0]-undec-7-ene
ETFAA - ethyl 4,4,4-trifluoro-3-oxo-butanoate
IVA - isovaleraldehyde, or 3-methyl-butanal
NMR - nuclear magnetic resonance
GLC - gas-liquid chromatography
% Assay - weight % desired product compound
% Yield - 100 x mols desired product / mol initial IVA starting material.
NOTE: Where a yield is shown herein in discussing the effect of varying a process parameter, all process variables not explicitly shown to be varied are held constant.

As outlined in Scheme I, preparation of diethyl 2-difluoromethyl-4-(2-methylpropyl)-6-trifluoromethyl-3,5-pyridinedicarboxylate is accomplished by a Hantzschtype base catalyzed intermolecular cyclization of ethyl 4,4,4-trifluoro-3-oxo-butanoate (ethyl trifluoroacetoacetate, or ETFAA) and isovaleraldehyde to form a substituted dihydroxypyran, followed by ammonolysis. Dehydration of the resultant dihydroxypiperidines gives a mixture of 1,4 and 3,4 dihydropyridine isomers. Dehydrofluorination of the dihydropyridines using an organic base such as DBU or 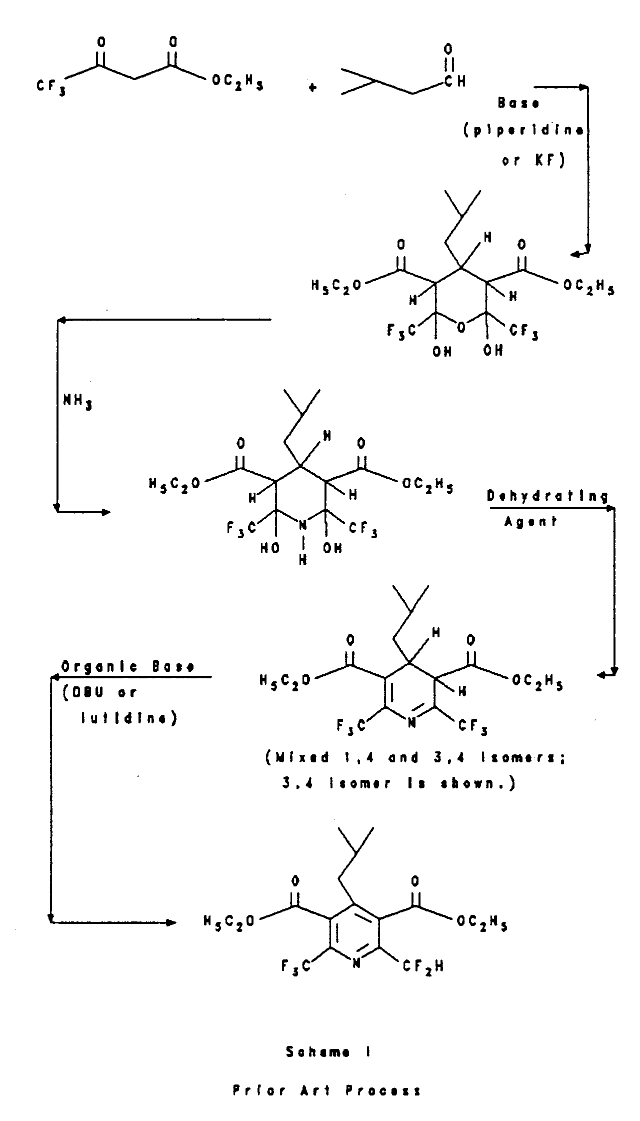
2,6-lutidine affords good yields (80% overall) of the pyridine diethylester. Examples 14 and 16 of U.S. Patent 4,692,184 are reproduced below in relevant part and are illustrative of the prior art.

### Example 14 OF U.S. Patent 4,692,184

### Preparation of dimethyl 2-(difluoromethyl)-6-(trifluoromethyl)-4-isobutyl-3,5-pyridinedicarboxylate

### (a) Dehydrofluorination Using DBU.

A mixture of 23.0 g (0.0591 mole) of the dihydropyridine, 12.2 g (0.077 mole) of 96% pure DBU, and 100 ml of THF is held at reflux for 3 days and poured into 250 ml of 3N HCl. The oil precipitate is extracted into ether (2x100 ml). The ether extracts are dried (MgSO₄) and concentrated to give 14.4 g of an oil which contained the desired product and acidic products. This oil is dissolved in ether and extracted with 100 ml of saturated sodium bicarbonate. The ether layer is dried (MgSO₄) and concentrated to give 8.9 g of an oil which is 71% pure desired product.

The sodium bicarbonate extract is acidified with concentrated HCl to give an oil which is extracted into ether. The ether layer is dried (MgSO₄) and concentrated to give 4.8 g of a residue which contained monocarboxylic acid and dicarboxylic acid (9:1) derived from the desired product. This residue is treated with 3.0 g (0.0217 mole) of potassium carbonate, 20 ml of methyl iodide, and 50 ml of acetone. The mixture is held at reflux for 42 hours and concentrated. The residue is treated with water and extracted with ether (2x100 ml). The ether layer is dried and concentrated. The residue is kugelrohr distilled at 1 torr (0.13 kilopascals) (pot temperature of 130°C.) to give 5.1 g (23.4% from the dihydropyridine) of the desired product. The 71% pure desired product described previously was chromatographed by HPLC using 3% ethyl acetate/cyclohexane as eluent to give an earlier fraction (0.79 g, retention time 7-8.5 min) which was identified as methyl 6-(difluoromethyl)-4-(isobutyl)-2-(trifluoromethyl)-3-pyridinecarboxylate. The second fraction is an additional 6.4 g (29.4%) of pure pyridine product.

### (b) Dehydrofluorination Using Tributylamine.

A mixture of 38.9 g of a 80% pure dihydropyridine and 20.5 g of tributylamine is heated to 155°C in 30 minutes. The reaction mixture was cooled to 30°C and diluted with 100 ml of toluene. The toluene solution is washed successively with 6N hydrochloric acid, saturated sodium bicarbonate, and brine, dried and concentrated to give 36.4 g of a 73% pure product which corresponds to a 85% yield. This reaction can also be carried out in excess of tributylamine (10 equivalent) giving essentially similar results.

### (c) Dehydrofluorination Using Tributylamine in Toluene.

A mixture of 38.9 g of 80% pure dihydropyridine, 20.4 of tributylamine and 30 ml of toluene is heated to 115°C in 40 minutes and held at 115°C for 1 hour and 40 minutes. The reaction mixture is cooled and worked up as in (b) to give 36.3 g of a 76% pure product which corresponds to a 90% yield.

### (d) Dehydrofluorination Using Triethylamine.

A mixture of 11.8 g of 80% pure dihydropyridine and 3.34 g of triethylamine is heated at 100°C for 10 minutes, then at 125°C for 10 minutes. The reaction mixture was cooled and worked up as in (b) to give 8.14 g of a 76% pure product which corresponds to a 63% yield.

### (e) Dehydrofluorination Using 2,6-Lutidine in the Presence of a Catalytic Amount Of DBU.

A mixture of 5.0 g of dihydropyridine and 2.13 g of 2,6-lutidine is heated at 143°C for 30 minutes. Two drops of DBU is added and the reaction mixture is heated for an additional one hour and 30 minutes, cooled and worked up as in (b) to give 4.23 g of the desired product. The reaction can also be carried out in excess of 2,6-lutidine and catalytic amount of DBU without solvent or in the presence of toluene as solvent giving similar results.

### EXAMPLE 16 of U.S. Patent 4,692,184

### Preparation of diethyl 2-(difluoromethyl)-4-isobutyl-6-(trifluoromethyl)-3,5-pyridinedicarboxylate

A mixture of 10.0 g (0.0240 mole) of diethyl 2,6-bis(-trifluoromethyl)-1, 4-dihydro-4-isobutyl-3,5-pyridinedicarboxylate, 3.65 g (0.0240 mole) of DBU and 150 ml of THF is held at reflux for 18 hours and concentrated. The residue is dissolved in ether and washed with diluted hydrochloric acid, dried (MgSO₄) and concentrated. The residue is kugelrohr distilled at 0.1 torr (0.01 kilopascals) to give 4.80 g (50%) of the desired product.

### DESCRIPTION OF THE INVENTION

According to the present invention there is provided a process for preparing a 4-(lower alkyl)-2-difluoromethyl-6-trifluoromethyl-3,5-pyridinedicarboxylic acid ester from a dihydropyridine starting material having the above substituents at the 3-, 4-, 5-, and 6- positions and having a trifluoromethyl substituent at the 2-position, which comprises contacting the starting material either with at least one half its molar amount of 1,4-diazabicyclo-(2.2.2)-octane (DABCO) or with a catalytic amount, for example about 0.01 to less than 0.5 of the molar amount of the dihydropyridine starting material, of 1,4-diazabicyclo-(2.2.2.)-octane and a sufficient amount of an additional base to insure substantially complete dehydrofluorination.

One embodiment of the present invention provides a process for preparing 2-difluoromethyl-6-trifluoromethyl-4-(2-methylpropyl)-3,5-pyridinedicarboxylic acid esters selected from dimethyl and diethyl esters from a dihydropyridine starting material having the above substituents at the 3-, 4-, 5-, and 6-positions and having a trifluoromethyl substituent at the 2-position, which comprises contacting the starting material either with at least one half its molar amount of 1,4-diazabicyclo-(2.2.2)-octane (DABCO) or with a catalytic amount of 1,4-diazabicyclo-(2.2.2)-octane with a sufficient amount of an additional base to insure complete dehydrofluorination.

In one embodiment of the present invention the amount of DABCO is about 1.0 times the molar amount of the dihydropyridine starting material.

In one embodiment of the present invention the DABCO is in the form of an aqueous solution. In this embodiment the process is preferably conducted in an inert aprotic solvent, preferably an inert aprotic solvent selected from benzene, toluene, xylenes, methylcyclohexane, monochlorobenzene and butyronitrile. Also, in this embodiment the process is preferably conducted in the substantial absence of molecular oxygen.

As in the Comparative Example above, the process of this invention is illustrated in detail below with reference to the preparation of the specific pyridine dicarboxylate compound prepared in Example 16 of U.S. Patent 4,692,184.

To improve yield of the desired pyridine dicarboxylate product, the following process of the present invention generally employs the same reaction steps as Scheme I. According to the present invention, the final step of the process of Scheme I, dehydrofluorination of the dihydropyridines prepared in the previous step to afford the final pyridine dicarboxylate product, is accomplished by treatment with DABCO in contrast to the prior art dehydrofluorination step which employs DBU or 2,6-lutidine as the organic base.

In this process step, DABCO may be employed in either stoichiometric or catalytic amounts. Because DABCO is a difunctional base, the stoichiometric DABCO method uses at least one half mol of DABCO per mol of starting IVA. Use of about one mol of DABCO is preferred. The catalytic DABCO method, on the other hand, employs substantially less DABCO such as about 0.01 to less than 0.50, and preferably about 0.05 to about 0.20 mol DABCO per theoretical mol of dihydropyridines (i.e., per mol of original IVA) in conjunction with an amount of an additional base which is adequate to effect substantially complete dehydrofluorination. The additional base used in the process in which DABCO is employed as a catalyst is a base selected from the group consisting of KOH, NaOH, K₂CO₃, Na₂CO₃, Ca(OH)₂, triethylamine, and tributylamine. Use of a catalytic amount of DABCO thus may result in a substantial economic benefit in the process.

Whichever dehydrofluorination method is employed, it is desirable to have some water present in the process to act as a solvent for salts (such as, for example, the hydrofluoride salt of DABCO and/or of the additional base if one is used) which may be formed in the process.

Whichever specific dehydrofluorination method is used, it is desirable to conduct this process step in the presence of an inert aprotic solvent. Such solvents include, but are not limited to, benzene, toluene, xylenes, cyclohexane, monochlorobenzene, butyronitrile, and like solvents. Moreover, while the temperature used in this process step is not particularly critical, it is preferred to use temperatures in the range of 50°C to 120°C, preferably 60°C to 90°C.

Using the stoichiometric DABCO dehydrofluorination method, the toluene solution from Step 2 is sparged vigorously with nitrogen to minimize oxidation byproducts. DABCO in an aqueous solution preferably at or near saturation in a ratio greater than 0.50 mol, and preferably about 1 mol per estimated mol of dihydropyridine is likewise sparged with nitrogen, and the two solutions are combined.

### Example 1

To a 3 L flask was added 502 g (1.2 mol) of diethyl 1,4-dihydro-2,6-bis(trifluoromethyl)-4-(2-methylpropyl)-3,5-pyridinedicarboxylate in 600 g of toluene. This solution was sparged subsurface with N₂ for 30 minutes. Subsequently, 146 g (1.3 mol) of DABCO and 219 g of H₂O were added as an aqueous solution. The reaction mixture was heated at 75-80°C for 4.75 hours while it was monitored for completion by GC. At completion of the reaction, the mixture was cooled to 50°C and the aqueous phase was removed. The toluene solution was washed with 130 g of a 15% brine solution, and the pH of the aqueous phase was adjusted to 4-5 with a small amount of concentrated sulfuric acid. The aqueous phase was then removed to leave a toluene solution of the desired product. Assay of the reaction mixture indicated the presence of 454 g (95%) of diethyl 2-difluoromethyl-4-(2-methylpropyl)-6-trifluoromethyl-3,5-pyridinedicarboxylate.

The experimental procedure above is representative of the procedure for the use of DABCO in the dehydrofluorination reaction. Additional examples utilizing different amounts of the base, solvents, and temperatures are included below. All materials were charged on the basis of the amount of dihydropyridine starting material used.

| Example | Mols DABCO | Solvent | Temp (°C) | Time (h) | Yield (%) |
|---|---|---|---|---|---|
| 2 | 1.1 | toluene | 90 | 2 | 100 |
| 3 | 1.1 | toluene | 70 | 6 | 95 |
| 4 | 1.1 | toluene | 50 | 7 | 93 |
| 5 | 1.5 | methylcyclohexane | 70 | 3 | 83 |
| 6 | 1.5 | CCl₄ | 70 | 3 | 73 |
| 7 | 1.25 | toluene/water | 70 | 2 | 86 |
| 8 | 1.0 | toluene/water | 90 | 3 | 86 |
| 9 | 0.6 | toluene/water | 80 | 3 | 85 |

While the process of this invention has been specifically illustrated in terms of a specific pyridine dicarboxylate product, it is equally applicable to the preparation of other pyridine compounds. Selection of the aldehyde starting material will, of course, determine the substituent at the 4-position of the final pyridine product. Likewise it is evident that lower alkyl trifluoroacetoacetate esters other than the ethyl ester may equally well be employed. Accordingly, the scope of this invention is to be limited only in accordance with the annexed claims.

## Claims

1. A process for preparing a 4-(lower alkyl)-2-difluoromethyl-6-trifluoromethyl-3,5-pyridinedicarboxylic acid ester from a dihydropyridine starting material having the above substituents at the 3-, 4-, 5-, and 6- positions and having a trifluoromethyl substituent at the 2-position, which comprises contacting the starting material either with at least one half its molar amount of 1,4-diazabicyclo-(2.2.2)-octane (DABCO) or with a catalytic amount of 1,4-diazabicyclo-(2.2.2)-octane and a sufficient amount of an additional base to insure substantially complete dehydrofluorination.

2. A process according to Claim 1 for preparing 2-difluoromethyl-6-trifluoromethyl-4-(2-methylpropyl)-3,5-pyridinedicarboxylic acid esters selected from dimethyl and diethyl esters from a dihydropyridine starting material having the above substituents at the 3-, 4-, 5-, and 6- positions and having a trifluoromethyl substituent at the 2-position, which comprises contacting the starting material either with at least one half its molar amount of 1,4-diazabicyclo-(2.2.2)-octane (DABCO) or with a catalytic amount of 1,4-diazabicyclo-(2.2.2)-octane with a sufficient amount of an additional base to insure complete dehydrofluorination.

3. A process according to Claim 1 or 2 wherein the amount of DABCO is about 1.0 times the molar amount of the dihydropyridine starting material.

4. A process according to Claim 1 wherein the catalytic amount of DABCO is about 0.01 to less than 0.5 of the molar amount of the dihydropyridine starting material.

5. A process according to Claim 1 or 2 wherein the DABCO is in the form of an aqueous solution.

6. A process according to Claim 5 wherein the process is conducted in an inert aprotic solvent.

7. A process according to Claim 6 wherein the aprotic solvent is selected from benzene, toluene, xylenes, methylcyclohexane, monochlorobenzene, and butyronitrile.

8. A process according to Claims 5 or 6 wherein the process is conducted in the substantial absence of molecular oxygen.

## Patentansprüche

1. Verfahren zur Herstellung eines Esters der 4-(Niederalkyl)-2-difluormethyl-6-trifluormethyl-3,5-pyridindicarbonsäure aus einem Dihydropyridin-Ausgangsmaterial mit den obigen Substituenten an den Positionen 3, 4, 5 und 6 und mit einem Trifluormethyl-Substituenten an der Position 2, bei welchem Verfahren das Ausgangsmaterial entweder mit mindestens der halben molaren Menge von 1,4-Diazabicyclo-(2.2.2)-octan (DABCO) oder mit einer katalytischen Menge von 1,4-Diazabicyclo-(2.2.2)-octan und einer ausreichenden Menge einer zusätzlichen Base in Kontakt gebracht wird, um eine im wesentlichen vollständige Dehydrofluorierung sicherzustellen.

2. Verfahren gemäß Anspruch 1 zur Herstellung von Estern der 2-Difluormethyl-6-trifluormethyl-4-(2-methylpropyl)-3,5-pyridindicarbonsäure, ausgewählt aus, dem Dimethyl- und Diethylester, aus einem Dihydropyridin-Ausgangsmaterial mit den obigen Substituenten an den Positionen 3, 4, 5 und 6 und mit einem Trifluormethyl-Substituenten an der Position 2, bei welchem Verfahren das Ausgangsmaterial entweder mit mindestens der halben molaren Menge von 1,4-Diazabicyclo-(2.2.2)-octan (DABCO) oder mit einer katalytischen Menge von 1,4-Diazabicyclo-(2.2.2)-octan und einer ausreichenden Menge einer zusätzlichen Base in Kontakt gebracht wird, um eine vollständige Dehydrofluorierung sicherzustellen.

3. Verfahren gemäß Anspruch 1 oder 2, wobei die Menge von DABCO etwa das 1,0fache der molaren Menge des Dihydropyridin-Ausgangsmaterials ist.

4. Verfahren gemäß Anspruch 1, wobei die katalytische Menge von DABCO etwa 0,01 bis weniger als 0,5 der molaren Menge des Dihydropyridin-Ausgangsmaterials ist.

5. Verfahren gemäß Anspruch 1 oder 2, wobei das DABCO in der Form einer wäßrigen Lösung vorliegt.

6. Verfahren gemäß Anspruch 5, wobei das Verfahren in einem inerten, aprotischen Lösungsmittel ausgeführt wird.

7. Verfahren gemäß Anspruch 6, wobei das aprotische Lösungsmittel aus Benzol, Toluol, Xylolen, Methylcyclohexan, Monochlorbenzol und Butyronitril gewählt ist.

8. Verfahren gemäß den Ansprüchen 5 oder 6, wobei das Verfahren praktisch bei Abwesenheit von molekularem Sauerstoff durchgeführt wird.

## Revendications

1. Procédé de préparation d'un ester d'un acide 4-(alkyl inférieur)-2-difluorométhyl-6-trifluorométhyl-3,5-pyridinedicarboxylique à partir d'une dihydropyridine de départ ayant les substituants ci-dessus sur les positions 3, 4, 5 et 6 et ayant un substituant trifluorométhyle en position 2, qui consiste à mettre en contact la matière de départ avec au moins la moitié de sa quantité en moles de 1,4-diazabicyclo[2.2.2]octane (DABCO) ou avec une quantité catalytique de 1,4-diazabicyclo[2.2.2]octane et une quantité d'une base additionnelle suffisante pour assurer une déshydrofluoration essentiellement complète.

2. Procédé selon la revendication 1 de préparation des esters de l'acide 2-difluorométhyl-6-trifluorométhyl-4-(2-méthylpropyl)-3,5-pyridinedicarboxylique, choisis parmi les esters diméthylique et diéthylique, à partir d'une dihydropyridine de départ ayant les substituants ci-dessus sur les positions 3, 4, 5 et 6 et ayant un substituant trifluorométhyle en position 2, qui consiste à mettre en contact la matière de départ avec au moins une moitié de sa quantité en moles de 1,4-diazabicyclo[2.2.2]octane (DABCO) ou avec une quantité catalytique de 1,4-diazabicyclo[2.2.2]octane et avec une quantité d'une base additionnelle suffisante pour assurer une déshydrofluoration complète.

3. Procédé selon la revendication 1 ou 2, dans lequel la quantité de DABCO est d'environ 1,0 fois la quantité en moles de la dihydropyridine de départ.

4. Procédé selon la revendication 1, dans lequel la quantité catalytique de DABCO est d'environ 0,01 à moins de 0,5 fois la quantité en moles de la dihydropyridine de départ.

5. Procédé selon la revendication 1 ou 2, dans lequel le DABCO se présente sous forme d'une solution aqueuse.

6. Procédé selon la revendication 5, dans lequel le procédé est mis en oeuvre dans un solvant aprotique inerte.

7. Procédé selon la revendication 6, dans lequel le solvant aprotique est choisi parmi le benzène, le toluène, les xylènes, le méthylcyclohexane, le monochlorobenzène et le butyronitrile.

8. Procédé selon la revendication 5 ou 6, dans lequel le procédé est mis en oeuvre essentiellement en l'absence d'oxygène moléculaire.
